# EUROPEAN PATENT APPLICATION

(11) **EP 1 757 936 A1**
(43) Date of publication of application: **28.02.2007**
(21) Application number: 05727746.9
(22) Date of filing: 29.03.2005
(51) Int. Cl.: G01N 33/544, G01N 33/576

(54) **SENSING TOOL**

(30) Priority: 31.03.2004 JP 2004104702
(71) Applicant: TORAY INDUSTRIES, INC., Tokyo 103-8666 (JP)
(72) Inventor: TANIZAWA, Katsuyuki, 5630214 (JP); KURODA, Shun'ichi, 5650872 (JP); JUNG, Giman;, Kamakura-shi,Kanagawa 2480034 (JP); AKIYAMA, Hideo;, Kanagawa 2480034 (JP); NOBUMASA, Hitoshi, 5200043 (JP)
(74) Representative: Kador & Partner
(86) International application number: PCT/JP2005/005803
(87) International publication number: WO 2005/095968

(57) **Abstract**

This invention relates to a hollow-nanoparticle-based biosensing tool, which comprises proteins capable of forming nanoparticles through the incorporation of a lipid bilayer and specific biorecognition molecules bound thereto and a biosensing method using such tool.

SEQUENCE LISTING

<110> Toray Industries INC.

<120> Sensing tool

<130> PH-2429-PCT

<140>
<141>

<150> JP 2004-104702
<151> 2004-03-31

<160> 4

<170> PatentIn Ver. 3.1

<210> 1
<211> 39
<212> DNA
<213> Artificial Sequence

<220>
<223> Inventor: Tanizawa, Katsuyuki
      Inventor: Kuroda, Shun'ichi
      Inventor: Jung, Giman
      Inventor: Akiyama, Hideo
      Inventor: Nobumasa, Hitoshi

<220>
<223> Description of Artificial Sequence: primer for the site-directed
      mutagenesis on pGLDLIIP39-RcT by QuikChange (registered mark)
      Site-Directed Mutagenesis

<400> 1
cgacaaggca tgggaggcgg ccgcagccct caggctcag                    39


<210> 2
<211> 39
<212> DNA
<213> Artificial Sequence

<220>

<223> Description of Artificial Sequence: primer for the site-directed
      mutagenesis on pGLDLIIP39-RcT by QuikChange (registered mark)
      Site-Directed Mutagenesis

<400> 2
ctgagcctga gggctgcggc cgcctcccat gccttgtcg                              39


<210> 3
<211> 65
<212> DNA
<213> Artificial Sequence

<220>
<223> Description of Artificial Sequence: primer for the GFP
      amplification by PCR, including six consecutive histidine
      codon sequence

<400> 3
agcggccgct caggttctca tcatcatcat catcatagtt ctggttcagt gagcaagggc 60
gagga                                                                   65


<210> 4
<211> 28
<212> DNA
<213> Artificial Sequence

<220>
<223> Description of Artificial Sequence: primer for the GFP
      amplification by PCR

<400> 4
gcggccgctc ttgtacagct cgtccatg                                          28

## Description

### Technical Field

The present invention relates to a nanoparticle-based biosensing tool, which comprises a lipid membrane and "a protein capable of forming particles" and which has a hollow construction. The present invention also relates to a sensing method.

### Background Art

In recent years, techniques for discerning and selectively sensing a specific molecule via labeling thereof or with the utilization of activity specific to such molecule have been actively studied. In particular, the need for a technique for sensing trace amounts of proteins, nucleic acids, compounds, and the like that are present *in vivo* or in the environment with high specificity and high sensitivity has been compelling in the fields of, for example, medicine, environmental inspection, food inspection, and life science for the purpose of early diagnosis of diseases or measurement of environmental hormones. Thus, research has been actively undertaken for techniques for specific labeling of substances or for improving measurement sensitivity.

Sensing techniques for trace amounts of substances with high sensitivity are roughly classified into two categories: techniques for sensing a target substance via labeling thereof with a fluorescence or luminescence; and techniques for sensing a target substance via, for example, surface plasmon resonance (SPR) or quartz crystal microbalance (QCM) that detects the binding to a target substance with high sensitivity. These techniques enable the sensing of substances in the ng to pg range. A sensing technique making use of fluorescence or luminescence labeling involves specific labeling of a substance to be detected with a substance that emits an intense strong signal such as fluorescence or luminescence (e.g., a green fluorescent protein or luciferase) and sensing of the labeled substance, thereby improving sensitivity ( Ohshima et al., Post-Sequence Protein Experimentation 2, Tokyo Kagaku Dojin Co., Ltd., 2002, pp. 130-146). In order to enhance detection sensitivity via such techniques, the application of nanoparticles has been actively studied in recent years. For example, a label substance (in particular, an enzyme such as HRP) is encapsulated in a liposome comprising a lipid and having a diameter of 200 to 500 nm, and a biorecognition molecule, such as an antibody, is allowed to covalently bind to the liposome surface with the aid of a crosslinking agent (JP Patent Publication (Kokai) No. 2003-149246 A). Also, an antibody or a biotin or streptavidin tag may be immobilized on the surface of a fluorescent particle having a diameter of 10 to 15 nm, which is referred to as "a quantum dot," to detect the target substance with the aid of fluorescence (US Patent No. 6274323 and Ozkan, M., Drug Discovery Today, 2004, vol. 9, pp. 1065-1071). In addition to these techniques, the following methods have been invented: a method involving the use of fine particles comprising polymers; a method wherein secondary antibodies are immobilized via some means on the surfaces of magnetic particles each having a diameter of approximately 100 nm produced by microorganisms and particle magnetism is used to detect as a labeled substance (Tsuyoshi Tanaka and Tadashi Matsunaga, the Journal of the Magnetics Society of Japan, 2004, vol. 28, pp. 675-679); a method involving the use of colloidal gold (Penn, S. et al., Current Opinion in Chemical Biology, 2003, vol. 7, p. p609-615); and other methods. For the purpose of the acceleration and simplification of measurements, an automated system has been developed that utilizes the suspension beads array (SBA) technique and is capable of simultaneous measurement of up to 100 types of antigens (Nolan, J. and Mandy, F., Cell and Molecular Biology, 2001, vol. 47, pp. 1241-1256). SPR is a technique for assaying binding strength or amounts of substances via measurements of changes in the charge density wave on a metal layer surface upon the binding of substances with the utilization of surface plasmons, which are electromagnetic waves localized at a metal/metal derivatives interface and propagate along the interface (Takayuki Okamoto and Ichiro Yamaguchi, Surface plasmon resonance and application thereof to laser microscope, the Review of Laser Engineering, the Laser Society of Japan, 1996, vol. 24, No. 10, pp. 1051-1053 and Ohshima et al., Post-Sequence Protein Experimentation 3, Tokyo Kagaku Dojin Co., Ltd., 2002, pp. 115-137). QCM is a technique that can be used to assay the binding level of a substance in the nanogram range and the speeds of binding and of dissociation with the utilization of a decreased number of basic oscillation frequency in proportion to the mass of substances adsorbed on the electrode (Ohshima et al., Post-Sequence Protein Experimentation 3, Tokyo Kagaku Dojin Co., Ltd., 2002, pp. 115-137 and Yoshio Okahata and Hiroyuki Furusawa, the Advanced Chemistry Series III, Maruzen, Co., Ltd., 2003, pp. 67-73).

Further, applications of a technique involving the presentation of proteins and the like at the cell surface to biosensing are examined. Specifically, methods of presenting functional proteins or peptides on the surfaces of living cells, such as yeast, or viruses have been attempted (Szardenings M, J., Recept Signal Transduct Res., 2003, 23, pp. 307-49; Mitsuyoshi Ueda, Bioscience and Industry, Japan Bioindustry Association, 1997, 55, pp. 253-254; Mitsuyoshi Ueda and Toshiyuki Murai, Bioengineering, the Society for Biotechnology, Japan, 1998, 76, pp. 506-510; JP Patent Publication (Kokai) No. 2001-316298 A; and JP Patent Publication (Kohyo) No. 2003-504506 A).

### Disclosure of the Invention

A large number of molecules with very important biological activities are present *in vivo* or in the environment in trace amounts (no larger than the pg range). Such trace amounts of molecules are difficult to assay via a sensing method represented by the aforementioned techniques, and thus, a step of amplifying sensed signals via concentration, purification, or the like is required. Such steps are complicated, and samples may be nonspecifically adsorbed to the apparatus during concentration or purification. This may result in loss of samples, or a large number of molecules in the samples may produce a masking effect. This makes it difficult to detect a target substance as the content of the target substance in samples becomes smaller. Accordingly, development of a method that is capable of amplifying sensed signals has been awaited in order to enable the sensing of trace amounts of molecules *in vivo* or in the environment. In addition, it is preferable if the technique for amplification is readily performed in combination with conventional sensing techniques (e.g., with the utilization of quantum dots, SPR, or QCM), which will be lead to enhanced sensitivity of conventional sensing techniques. Also, a technique for amplifying sensed signals has been awaited as an indispensable technique when quantitative and highly sensitive sensing of trace amounts of samples is intended, as with the case of biochips typified by DNA or protein chips.

As an effective measure for attaining the object of enhancing sensitivity for biosensing, a biorecognition molecule that specifically senses the target substance is allowed to bind to the surface of a structure that is easily detectable. The structure, which has been bound to the target molecule, is then sensed, and thus, sensed signals can be amplified. Examples of structures that can be easily sensed include particles such as quantum dots, liposomes, polymers, magnetic particles, or colloidal gold, and living cells such as yeast, and viruses.

The exclusive label substance of quantum dots is fluorescence. Accordingly, quantum dots are disadvantageous in terms of versatility; i.e., most common enzymes, such as horse radish peroxidase (HRP) or alkaline phosphatase (AP), in immunological detection that involves the use of antibodies as biorecognition molecules, cannot be used as label substances. Thus, use of an expensive detector is required in order to detect quantum dot fluorescence. Also, quantum dots are very expensive. In the case of a technique involving the use of a liposome, enzymes such as HRP or AP with sensitivity higher than that of fluorescence may be used. In such a case, however, such enzymes must be enclosed in particles, and this requires performance of harsh processes that are likely to cause proteins to become denatured, such as high-temperature treatment, vortex, or ultrasonication. Since this technique requires separate synthesis and purification of labeling enzymes and addition thereof, the production cost is high, as with the case of quantum dots. The use of liposomes is also disadvantageous in terms of low physical stability. Also, labeling techniques using magnetic particles or colloidal gold are limited, and the detection sensitivity thereof is insufficient. Many fine particles comprising polymers or metals are autofluorescent materials, and such particles are likely to develop a high-level background at the time of sensing of fluorescence or luminescence with the use of an optical detector. Thus, the selection of such materials involves many restrictions. Existing techniques for amplifying detected signals (i.e., sensing techniques) suffer from various issues that involve disturbance of the practical applications thereof, in terms of sensitivity, versatility, production cost, and the necessity for an expensive detector, for example.

In addition to such drawbacks, all the existing techniques for amplifying detected signals suffer from increased nonspecific binding arise from biorecognition molecules that become randomly bound to the particles, for the following reason. When biorecognition molecules such as antibodies are bound to particles, it becomes very difficult for biorecognition molecules to bind to particle surfaces in an aligned state. Such increase of nonspecific binding would deteriorate detection sensitivity. In particular, nonspecific binding becomes a more serious issue of concern as the quantity of the target substance becomes smaller in the sample. In order to prevent such nonspecific binding, particles may be covered with lipids at sites other than the sites where the particles are bound to the target substances. For example, particles comprising artificial or organism-derived lipid membranes can be used in order to prevent nonspecific binding. Artificial lipid particles (liposomes) would suffer from the disadvantages, such as small amounts of biorecognition molecules to be presented and difficulties in aligning the biorecognition molecules, in addition to the aforementioned drawbacks.

The existing techniques for amplifying detected signals also involve the following issue. That is, particles, label substances, and biorecognition molecules must be separately prepared and these members must be artificially bound to one another via crosslinking or the like. Thus, quality control is difficult, and productivity involves issues of concern such as quality variation created per production batch. Organic solvents and the like are often used when producing particles that are used for such existing techniques for amplifying detected signals, which would affect the environment. Thus, existing techniques for amplifying detected signals suffer from difficulties such as those related to detection specificity or accuracy, productivity, or environmental safety.

While living cells or viruses can present biorecognition molecules aligned on membrane surfaces via genetic engineering or other means (Microbiology and Molecular Biology Reviews, 1171-1190, 1998), the application thereof is tightly restricted in view of safety, such as in terms of infection or environmental impacts. Further, when living cells are used, endogenous enzymes or compounds may increase background levels or disturb the sensing reaction. Also, substances other than the target substance may undergo nonspecific binding to the surface structure (in particular, to cell walls) and generate high background levels.

As disclosed in JP Patent Publication (Kokai) No. 2001-316298 A, "proteins capable of forming particles" incorporate the lipid bilayer in a cell via intracellular self-organization and form hollow nanoparticles. On the surfaces of such hollow nanoparticles, self-organized "proteins capable of forming particles" are aligned at high density.

Up to the present, there has been no example of application of the sensing technique using such particles for amplification of signals or enhancement of sensitivity. Applications of such sensing technique are limited to the identification of the occurrence of virus infection via detection of such particles or DDS research for organ-specific transportation of drugs, for example.

Under such circumstances, the present invention is directed to resolving the drawbacks of conventional techniques and to providing a versatile tool that is capable of sensing a target substance (e.g., a gene, protein, or compound) with high accuracy and high sensitivity and a technique for sensing using such tool.

The present inventors have conducted concentrated studies in order to attain the above objects. Consequently, they have found that the use of the hollow nanoparticles results in the solution of all the drawbacks of the existing sensing techniques that require high sensitivity. This has led to the completion of the present invention.

Specifically, upon alteration or modification of the "proteins capable of forming particles" via genetic engineering or other means, such proteins can bind and align biorecognition molecules, such as antibodies, or label substances on particle surfaces at high density. Thus, the sensitivity for detecting such particles is dramatically enhanced.

Further, the most important feature of the hollow nanoparticles according to the present invention is that nonspecific binding is less likely to occur because of a constitution such that the lipid bilayer surrounds the self-organized "proteins capable of forming particles" and such that the particles are covered with lipids at sites other than the sites where the particles are bound to the target substances. Thus, detection sensitivity and accuracy are dramatically enhanced.

In addition to the fact that a wide variety of biorecognition molecules can be bound to particle surfaces, a fluorophore, an enzyme, a radioisotope, and the like can be used as particle-labeling substances. Thus, the versatility of the particles of the present invention is extensive. When the hollow nanoparticles of the present invention are produced from cells, biorecognition molecules, such as antibodies, label enzymes, and the like can be simultaneously produced via genetic engineering techniques, cloning enables constant production of hollow nanoparticles with uniform quality, and easily handleable yeast can be employed for a particle production system. Thus, such technique is excellent in terms of productivity or production cost. Since enzymes or the like can be used as label substances, an expensive detector is not always required. Since the particles of the present invention are produced from cells, use of organic solvents or the like is not required during the production, such particles are biodegradable particles composed of proteins and lipids, and such particles are thus excellent in terms of environmental safety. Further, such particles are free of the risk of infection or the like, the detection would not be disturbed by endogenous enzymes or compounds, and generation of background noises can be inhibited, since the technique for producing such particles does not involve the use of living cells or viruses. Furthermore, the particles of the present invention are not autofluorescent, they are physically stable, and they can maintain their configurations even in the presence of heat, a surfactant, or 8M urea.

In order to solve the above problems, a first embodiment of the present invention is a sensing tool comprising "proteins capable of forming nanoparticles" through the incorporation of a lipid bilayer and biorecognition molecules bound thereto.

A second embodiment of the present invention is the sensing tool according to the first embodiment, wherein the proteins are virus surface antigen proteins.

A third embodiment of the present invention is the sensing tool according to the first or second embodiment, wherein at least one type of molecule selected from the group consisting of fluorescent, luminescent, light absorptive, and radioisotope molecules is bound to the lipid bilayer of the hollow nanoparticles, the proteins capable of forming particles, or biorecognition molecules, or is enclosed in the hollow nanoparticles.

A fourth embodiment of the present invention is a biosensing tool using a flat membrane-like array of biorecognition molecules, which comprises the hollow nanoparticles according to the first, second, or third embodiment aligned on a substrate.

A fifth embodiment of the present invention is a biosensing method using the sensing tool according to any of the first to fourth embodiments.

The present invention provides a versatile tool for effectively detecting trace components *in vivo* or in the environment with the utilization of hollow nanoparticles presenting biorecognition molecules and a sensing method using such tool. With the utilization of such tool and method, biorecognition molecules, such as antibodies, or label substances can be bound to and aligned on particle surfaces upon alteration or modification of the "proteins capable of forming particles" via genetic engineering or other means at high density. Thus, the sensitivity of detection of such particles is dramatically enhanced.

The most important feature of the hollow nanoparticles according to the present invention is that nonspecific binding is less likely to occur because of a constitution such that the lipid bilayer surrounds the self-organized "proteins capable of forming particles" and such that the particles are covered with lipids at sites other than the sites where the particles are bound to the target substances. Thus, detection sensitivity and accuracy are dramatically enhanced.

In addition to the fact that a wide variety of biorecognition molecules can be bound to particle surfaces, a fluorophore, an enzyme, a radioisotope, and the like can be used as particle-labeling substances. Thus, the versatility of the particles of the present invention is extensive. When the hollow nanoparticles of the present invention are produced from cells, biorecognition molecules, such as antibodies, label enzymes, and the like can be simultaneously produced via genetic engineering techniques, cloning enables constant production of hollow nanoparticles with uniform quality, and easily handleable yeast can be employed for a production system of the particles. Thus, such technique is excellent in terms of productivity and production cost. Since enzymes or the like can be used as label substances, an expensive detector is not always required. Since the particles of the present invention are produced from cells, use of organic solvents or the like is not required during production, such particles are biodegradable particles composed of proteins and lipids, and such particles are thus excellent in environmental safety. Further, such particles are free of the risk of infection or the like, detection would not be disturbed by endogenous enzymes or compounds, and generation of background noise can be inhibited, since the technique for producing such particles does not involve the use of living cells or viruses. Furthermore, the particles of the present invention are not autofluorescent, and they are physically stable. Further, the tool and the method according to the present invention can be applied to existing techniques for assaying substances, such as SPR, QCM, or a quantum dot method, and thus the industrial applicability thereof is high.

### Brief Description of the Drawings

Fig. 1 schematically shows a protein region of the HBsAg gene employed in the examples of the present invention, wherein numeral references 1 to 8 each independently represent functions of a site of the surface antigen.
Fig. 2 schematically shows examples of the expression of the HBsAg particles with the use of genetically recombinant yeast used in the examples of the present invention and the procedure for purifying the same ((a) preparation of genetically recombinant yeast; (b) culture in High-Pi medium; (c) culture in 8S5N-P400 medium; (d) grinding; (e) density gradient centrifugation; and (f) HBsAg particles).
Fig. 3 shows the effects of the HBsAg particles used in the examples of the present invention for amplifying SPR signals, wherein the x axis represents the duration of reaction (sec) and the y axis represents signal intensity.
Fig. 4 schematically shows pGLDLIIP39-RcT GFP used in the examples of the present invention. Components of this diagram except for the site at which His6-GFP is inserted into the *Not*I restriction enzyme are the same as those of Fig. 1.
Fig. 5 shows the results of assaying the effects of the GFP-fused particles used in the examples of the present invention on the amplification of the sensed signals via SPR, wherein the x axis represents the duration of reaction (sec) and the y axis represents signal intensity.
Fig. 6 shows the effects of the GFP-fused particles used in the examples of the present invention on the inhibition of nonspecific binding of a flat membrane-like array of biorecognition molecules and the effects of aligning biorecognition molecules, wherein the x axis represents the duration of reaction (sec) and the y axis represents signal intensity.

### Description of reference numerals in the drawings

- 1:: Inhibition of emission
- 2:: Receptor
- 3:: Sugar chain 1
- 4:: Receptor for polymerized serum albumin
- 5:: Transmembrane
- 6:: Stabilization
- 7:: Sugar chain 2
- 8:: Transmembrane, oligomerization, secretion

This description includes part or all of the contents as disclosed in the description of Japanese Patent Application No. 2004-104702, which is a priority document of the present application.

### Preferred Embodiments of the Invention

The present invention concerns a sensing tool comprising proteins capable of forming nanoparticles through the incorporation of a lipid bilayer and biorecognition molecules bound thereto.

Preferably, the present invention concerns a sensing tool, wherein the biorecognition molecules are covalently bound to the proteins.

Preferably, the present invention concerns a sensing tool, wherein the nanoparticles are hollow nanoparticles.

Preferably, the present invention concerns a sensing tool, wherein the proteins are virus surface antigen proteins.

Preferably, the present invention concerns a sensing tool, wherein the proteins are hepatitis B virus surface antigen proteins.

Preferably, the present invention concerns a sensing tool, wherein the proteins are capable of forming nanoparticles through the incorporation of a lipid bilayer derived from eukaryotic cells.

Preferably, the present invention concerns a sensing tool, wherein the proteins are capable of forming nanoparticles through the incorporation of a lipid bilayer derived from yeast.

Preferably, the present invention concerns a sensing tool, wherein the proteins are capable of forming nanoparticles through the incorporation of a lipid bilayer derived from animal or insect cells.

Preferably, the present invention concerns a sensing tool, wherein the biorecognition molecules are molecules that control cellular functions.

Preferably, the present invention concerns a sensing tool, wherein the biorecognition molecules are antigens, antibodies, parts of antibodies, or antibody analogues.

Preferably, the present invention concerns a sensing tool, wherein the biorecognition molecules are cell surface or intracellular receptor proteins that bind to ligand substances, mutants thereof, parts thereof, or substances bound thereto.

Preferably, the present invention concerns a sensing tool, wherein the biorecognition molecules are enzymes, mutants thereof, parts thereof, or substances bound thereto.

Preferably, the present invention concerns a sensing tool, wherein at least one type of molecule selected from the group consisting of fluorescent, luminescent, light absorptive, and radioisotope molecules is bound to the biorecognition molecules.

Preferably, the present invention concerns a sensing tool, wherein at least one type of molecule selected from the group consisting of fluorescent, luminescent, light absorptive, and radioisotope molecules is bound to the proteins capable of forming particles.

Preferably, the present invention concerns a sensing tool, wherein at least one type of molecule selected from the group consisting of fluorescent, luminescent, light absorptive, and radioisotope molecules is bound to the lipid bilayer.

Preferably, the present invention concerns a sensing tool, wherein at least one type of molecule selected from the group consisting of fluorescent, luminescent, light absorptive, and radioisotope molecules is enclosed in the hollow nanoparticles.

Preferably, the present invention concerns a sensing tool, which employs a flat membrane-like array of biorecognition molecules comprising nanoparticles aligned on a substrate.

The present invention concerns a biosensing method, which involves the use of a sensing tool comprising proteins capable of forming nanoparticles through the incorporation of a lipid bilayer and biorecognition molecules bound thereto.

In the present invention, the term "lipid bilayer" refers to a membrane having a thickness of 5 to 20 nm and composed of two lipid layers. The polar head group of amphipathic lipid is in contact with a hydrophilic solvent in each layer, and a nonpolar hydrocarbon group faces inward the bilayer structure. Examples of lipid bilayers include biomembranes such as cell membranes, nuclear membranes, membranes of the endoplasmic reticulum, Golgi membranes, and vacuolar membranes of living cells and liposomes that are artificially produced. A lipid bilayer derived from membranes of the endoplasmic reticulum is particularly preferable. A lipid bilayer is preferably derived from eukaryotic cells, such as animal, plant, fungal, and insect cells, and a yeast-derived lipid bilayer is particularly preferable.

In the present invention, the term "recognition" refers to specific binding of two or more substances in accordance with the structures or properties thereof. Such binding is carried out via any intermolecular interactions, such as covalent binding, ionic binding, hydrophobic binding, hydrogen binding, or metal binding. Even if the substance to be recognized is contained among contaminants, such specific binding can be carried out.

In the present invention, the configuration of "biorecognition molecules bound to proteins capable of forming nanoparticles" is not particularly limited. Preferably, biorecognition molecules are covalently fused to "proteins capable of forming particles" via genetic engineering (i.e., peptide binding); i.e., the biorecognition molecules and the "proteins capable of forming particles" form particles while being expressed in cells as fusion proteins. Alternatively, the biorecognition molecules are bound to the "proteins capable of forming particles" via, for example, physical or chemical modification or adsorption. "Physical or chemical" modification or adsorption refers to, for example, modification or adsorption via ionic binding, hydrophobic binding, hydrogen binding, metal binding, covalent binding such as disulfide binding, or a combination of any of such bindings.

A plurality of biorecognition molecules may be bound to a "protein capable of forming particles." Also, a plurality of biorecognition molecules may together form a single biorecognition molecule. In such a case, biorecognition molecules can be bound to each other without particular limitation. Such binding may be carried out via covalent binding, ionic binding, hydrophobic binding, hydrogen binding, metal binding, or a combination of any of such bindings.

In the present invention, the term "hollow nanoparticles" refers to nanosize particles preferably having a diameter of 20 nm to 500 nm, more preferably of 50 nm to 200 nm, and further preferably of 80 nm to 150 nm, the insides of which have spaces that can contain a variety of substances (e.g., a fluorescent dye, protein, nucleic acid, or compound). Such spaces are not necessarily gaseous spaces, and such spaces are preferably liquid spaces that can stably preserve the substances therein.

In the present invention, the term "ligand substances" refers to substances that are bound to relevant receptors such as hormones (molecules that allow a cell located at a given site of an individual organism to communicate with a cell located at another site), growth factors (substances that regulate cell growth), or neurotransmitters (substances that transmit neural information through the synapse) and are involved in intracellular or intercellular communications. Such ligand substances comprise peptides, protein, or steroidal or low-molecular-weight compounds.

In the present invention, the term "cell surface" refers to the cell wall or the inside or surface of the cell membrane. The ligand substances may be obtained by, for example, a method wherein ligand substances are purified from living cells, or a method wherein ligand-expressing plasmids are prepared via genetic recombination, and ligand substances are produced with the use of such plasmids in cells such as *E. coli,* yeast, insect, animal, or plant cells or in a cell-free protein synthesis system, followed by purification, or via chemical synthesis.

In the present invention, the term "intracellular" refers to all parts enveloped by a cell membrane. The term "receptor proteins" refers to proteins that bind to ligand substances and that are involved in intracellular or intercellular communications. Examples thereof include: proteins such as guanine nucleotide-binding proteins or intranuclear receptors, which are present on the cell membranes or in the cells, which are bound to ligand substances such as hormones, and which transmit signals from the exteriors of the cells to their interiors or from the cytoplasms to the nucleus; receptor tyrosine kinases to which growth factors are bound as ligand substances; and proteins that recognize neurotransmitters such as adrenergic receptors and transmits the signals thereof.

In the present invention, the term "receptor proteins" also refers to proteins that are present in a cell membrane and involved in active transportation of metal ions and the like. Such "receptor proteins" may be obtained by, for example, a method wherein ligand substances are purified from living cells, or a method wherein ligand-expressing plasmids are prepared via genetic recombination, and ligand substances are produced with the use of such plasmids in cells such as *E. coli,* yeast, insect, animal, or plant cells or in a cell-free protein synthesis system, followed by purification, or via chemical synthesis. In the present invention, a "receptor protein" is not necessarily a full-length sequence. Such receptor protein may be a variant sequence formed by amino acid substitution, deletion, or addition or part of a receptor protein, as long as it is capable of specifically binding to a ligand.

In the present invention, the term "enzymes" refers to proteins that control the vital phenomena of cells via modification, cleavage, fusion, denaturation, or binding of nucleic acids, sugars, lipids, or other proteins intracellularly and extracellularly. Examples thereof include protease, phosphatase/sugar chain modifying enzymes, nucleic acid cleavage (restriction) enzymes, glycolytic/lipidolytic enzymes, and nucleic acid/protein/sugar/lipid biosynthetases. The term "enzymes" also refers to coenzymes that are inactive by themselves but assist the activities of other enzymes.

In the present invention, the term "variants" of the receptor proteins or enzymes refers to: variants formed by introducing substitution of a single point or plurality of amino acids into proteins via genetic engineering techniques; a variant derived from a full-length protein by partial deletion or addition of a new protein sequence; and a variant formed by the application of modification to a part of a protein with a nucleic acid, sugar, lipid, or a compound and having activity of recognizing the same molecule that is recognized by a receptor protein or enzyme before variation as a biorecognition molecule. In order to prepare such variant, 12 to 18 PCR cycles are carried out using primers for performing amino acid deletion, substitution, or addition on a cyclic plasmid containing a gene expressing such protein, and using mutation kits such as the QuikChange site-directed mutagenesis kit, the QuikChange multi site-directed mutagenesis kit, or the QuikChange XL site-directed mutagenesis kit (Stratagene), the PCR product is cleaved with the *Dpn*I restriction enzyme, and the resultant is transformed into *E. coli.* Specific examples of modification to a part of a protein with a nucleic acid, sugar, lipid, or compound include phosphorylation of serine or threonine in a protein with phosphoenzyme, sugar chain addition to asparagines, serine, or threonine by a glycosylating enzyme, and reduction-alkylation of a cysteine residue with the use of a reduction/alkylation reagent. In order to prepare such products, phosphoric acid, sugar chain, or the like is mixed with a protein, and a phosphoenzyme or glycosylating enzyme is added to maintain the conditions optimal for such enzymes (e.g., temperature, pH, and salt concentration). Also, such products may be obtained by introducing a reducing agent, such as dithiothreitol or mercaptoethanol, into a protein-containing solution to bring the final concentration to 5 mM, performing reduction reactions at around 60°C at neutral or higher pH levels for 1 hour, and further adding an alkylation agent, i.e., 5 mM to 15 mM iodoacetamide, to perform reactions at room temperature for at least 1 hour. It should be noted that various other types of modification could be applied to a protein, in addition to the aforementioned modifications.

In the present invention, a "part" of a receptor protein or enzyme refers to a partial amino acid sequence having all or some properties of such receptor protein or enzyme (a sequence comprising at least five consecutive amino acids of the full sequence) that is derived or synthesized from the full sequence via a genetic or protein engineering technique. Such partial sequence has activity of recognizing the same target substance that is recognized by the entire receptor protein or enzyme.

The term "substance that binds to" a receptor protein or enzyme used in the present invention refers to a substance that specifically binds to an active site of a receptor protein or enzyme, such as a ligand substance for a receptor protein or a substrate or inhibitor for an enzyme. This term also refers to a substance that is constantly bound to such protein in the cells or a substance that binds to a protein to maintain the structure of the protein or assist or supplement the activity thereof. Examples of such substance include a coenzyme, such as a metal ion, that assists the activity of a molecular chaperone protein or enzyme in the intranuclear receptor and membrane proteins that play a role in adequate transportation of a receptor protein or enzyme to a given site in the cells or in immobilization of a membrane-bound receptor protein or enzyme to a membrane.

In the present invention, the term "proteins having fluorescent, luminescent, light absorptive, and radioisotope molecules or derivatives thereof' refers to: a green fluorescent protein or a variant thereof that emits fluorescence upon reception of a light of given wavelength; a protein that comprises a fluorescent compound bound thereto via chemical modification; a protein having activity of allowing a substrate, such as luciferase, HRP, or AP, to emit light or a protein comprising such protein bound thereto; a protein exhibiting an intense absorption at a given wavelength, such as a hemoprotein; a protein comprising a light-absorbing compound bound thereto; a protein comprising a light-absorbing peptide or protein bound thereto; a protein having activity of allowing a substrate, such as galactosidase, HRP, or AP, to develop a color or a protein comprising such protein bound thereto; and a protein labeled with a radioisotope, such as H3, C14, N15, P32, S35, Co57, Se75, or I125, via the addition thereof to the medium at the time of protein expression or via chemical modification. A derivative of such protein refers to a product prepared by cleaving part of a protein via a genetic or protein engineering technique and allowing the cleavage product to covalently bind to the other molecule, i.e., a nucleic acid, sugar chain, lipid, other protein, or compound. The term "protein derivative" also refers to a covalent protein-compound complex prepared via chemical modification or with the use of an enzyme and a protein modified with a sugar chain, nucleic acid, or lipid. In such a case, fluorescent, luminescent, light absorptive, or radioisotope molecules may be labeled with a nucleic acid, sugar chain, lipid, or the like that is bound to a protein as its derivative, instead of the protein itself.

In the present invention, a fluorescent "compound" refers to a substance that absorbs light at a given wavelength and emits fluorescence in a wavelength range different from the absorbed wavelength. Examples thereof include fluorescein, rhodamine, dansyl, Lucifer Yellow VS, umbelliferyl, rare-earth chelate, Cy2, Cy3, Cy5, fluorescein isothiocyanate (FITC), Alexa® (Molecular Probe), and quantum dot. In the present invention, the "substrate" is not particularly limited, as long as the substrate is made of a metal, plastic, or organic or inorganic polymer material. Preferable examples thereof include: resins such as polystyrene, polyethylene, polypropylene, polymethylpentene, polymethylmethacrylate (PMMA), polycarbonate (PC), polysulfone, polytetrafluoro-Teflon (PVDF), cellulose, silicon, mica, polymethylpentene (PMP or TPX®), polystyrene (PSt), polytetrafluoroethylene (PTFE), ABS, and polydimethylsiloxane (PDMS) resins; copolymers or composites comprising the aforementioned high-molecular-weight resin compounds; glasses and glass composites such as quartz glass, Pyrex® glass, soda glass, borate glass, silicate glass, and borosilicate glass; metals and metal composites such as gold, silver, copper, nickel, and cobalt; and ceramics and ceramic composites. Also, use of a substrate, the entire surface or at least a portion subjected to sensing, covered with such material is preferable. Such substrate materials can be used in combinations of two or more. For example, use of a glass substrate covered with a metal or a resin substrate covered with a metal is preferable. The term "substrate" of the present invention includes substrates with surfaces of that are subjected to coating or grafting with a hydrophilic polymer (e.g., polyethylene glycol or polyvinyl alcohol), hydrophobization, or radical addition, or a substrate covered, modified, or treated with a protein such as an antibody, a nucleic acid such as DNA, or sugar.

The term "flat membrane-like array of biorecognition molecules" used in the present invention refers to a substrate covered with particles via the binding of hollow nanoparticles, defined in the present invention, to a substrate. Such array can be prepared by, for example, a method wherein hollow nanoparticles are physically adsorbed to the substrate surface or a method wherein a substrate is previously modified with a protein, peptide, nucleic acid, sugar chain, lipid, or metal to which the hollow nanoparticles of the present invention are specifically bound to thereby realize specific binding between proteins, lipids, or sugar chains on the hollow nanoparticle surfaces and a substrate. In the "flat membrane-like array of biorecognition molecules" of the present invention, hollow nanoparticles are bound to a substrate in a particulate state or particles are first bound to a substrate and then ground via dehydration, ultrasonication, or the application of electric shock to form a flat membrane, and the resultant is bound to a substrate, according to need. Accordingly, the thickness of the "flat membrane-like array of biorecognition molecules" is 5 nm to 500 nm. The "flat membrane-like array of biorecognition molecules" of the present invention is characterized in that the particles of the present invention are formed via self-organization of the "proteins capable of forming particles." Thus, biorecognition molecules bound to the "proteins capable of forming particles" are regularly aligned on the surface of the "flat membrane-like array of biorecognition molecules" formed by particles at high density. Thus, the "flat membrane-like array of biorecognition molecules" is suitable in terms of detection accuracy and sensitivity. In addition, the surface of the "flat membrane-like array of biorecognition molecules" of the present invention is composed of a lipid bilayer at portions other than the portions composed of "proteins capable of forming particles." Advantageously, the "flat membrane-like array of biorecognition molecules" is characterized in that nonspecific binding of contaminants that are not involved in the reactions is less likely to occur.

With the utilization of the sensing tool for detecting trace components according to the present invention, which comprises lipids, proteins, and hollow nanoparticles, and the sensing method using such tool, biorecognition molecules are allowed to bind to proteins capable of forming particles to realize significant amplification of signals detected and to specifically recognize various target molecules. This enables detection of trace components *in vivo* or in the environment that had been difficult or impossible to detect in the past.

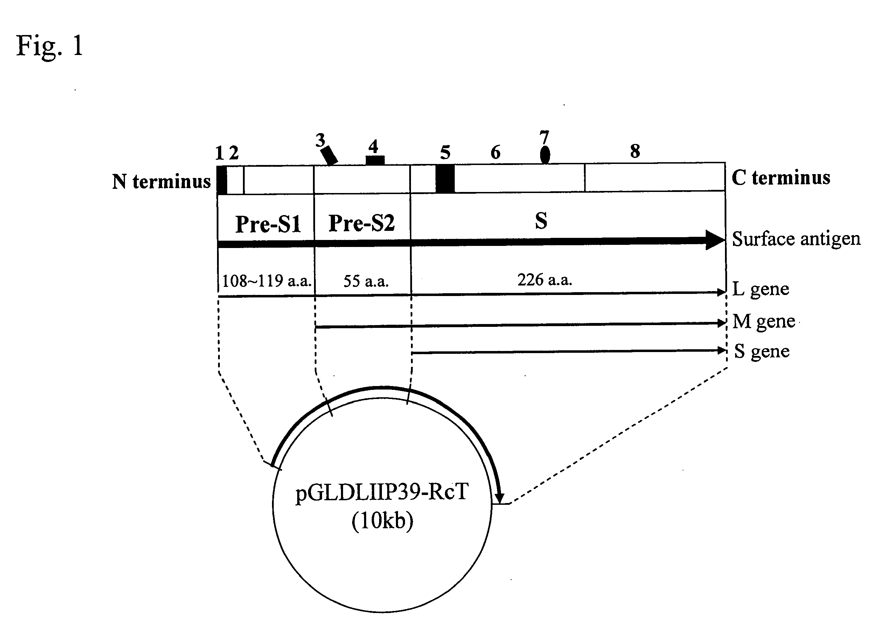

The term "sensing tool" refers to a means for detecting changes in, for example, fluorescence, luminescence, light absorption, or radiation intensity by immobilizing target substances on a substrate and binding hollow nanoparticles comprising biorecognition molecules that recognize the target substances bound thereto. The term "sensing tool" also refers to a means for detecting changes in, for example, turbidity, fluorescence, luminescence, or light absorption by adding hollow nanoparticles comprising biorecognition molecules that recognize the target substances bound thereto to a solution containing the target substances. The term "sensing tool" of the present invention further refers to a means for detecting target substances by the sandwich technique, wherein the hollow nanoparticles are immobilized on a substrate to constitute a "flat membrane-like array of biorecognition molecules" and the target substances are bound thereto. Alternatively, the target substances are bound while the particles are immobilized in the solution or on the substrate, and particles having other biorecognition molecules that recognize the target substances are allowed to react with the target substances. The term "sensing tool" of the present invention also includes products of various types of modification for the purpose of further enhancement of detection sensitivity. An example of modification is the use of hollow nanoparticles that comprise an antibody that recognizes the target substance immobilized on a substrate and another antibody that specifically recognizes the former antibody bound thereto and that present on the surfaces thereof or enclose fluorescent, luminescent, light absorptive, or radioisotope molecules. As exemplified in Fig. 1, the hollow nanoparticles used in the biosensing tool according to the present invention can be prepared in the following manner. That is, an expression vector is first prepared, which has a promoter sequence that can express proteins in cells at a high level, initiator codons for proteins downstream thereof, a signal protein sequence for transporting a protein to the endoplasmic reticulum and a sequence of a "protein capable of forming particles" ligated immediately downstream thereof, and a biorecognition molecule sequence introduced between the signal protein sequence and the sequence of a "protein capable of forming particles," in the sequence of a "protein capable of forming particles," or downstream of the sequence of a "protein capable of forming particles." The resulting expression vector is then introduced into the cells to induce the expression of the hollow nanoparticles, followed by purification.

Expression and purification of hollow nanoparticles using yeast are described in Examples 1 and 2. Hollow nanoparticles are produced from any cells without particular limitation as long as such cells have a lipid bilayer, and particularly preferably have an endoplasmic reticulum. Preferably, hollow nanoparticles are prepared from eukaryotic cells, such as animal, plant, fungi, or insect cells. Yeast cells are particularly preferable because of the following properties. That is, yeast is easily handleable, it can be easily recombined, and protein expression efficiency is satisfactory.

The hollow nanoparticle-based biosensing tool according to the present invention can comprise: a "substrate" to which the samples, i.e., the target substances to be sensed, are to be bound; "hollow nanoparticles" comprising biorecognition molecules, which specifically detect the target substances from among the samples bound to the substrate and specifically recognize the target substances for amplifying the detected signals, bound thereto and presenting on the surfaces or enclose fluorescent, luminescent, or light absorptive, or radioisotope molecules; and a "detector" that can detect the level of fluorescence, luminescence, light absorption, or radioisotope derived from the hollow particles. A sensing technique using such sensing tool can be achieved by binding samples to the substrate, allowing the hollow nanoparticles to react thereon, rinsing off the hollow nanoparticles that did not bind to the target substances to be sensed, and detecting the level of fluorescence, luminescence, light absorption, or radioisotope emitted from the hollow nanoparticles that have remained on the substrate (i.e., the hollow nanoparticles that specifically bound to the target substances to be sensed) using a detector. Samples can be bound to a substrate by, for example, a method wherein samples in the form of a dehydrated powder or a suspension or solution are allowed to react with the substrate to induce physical adsorption or a method wherein a substrate is modified with a protein, peptide, nucleic acid, sugar chain, lipid, metal, or flat membrane-like array of biorecognition molecules in advance to specifically bind the target substance to be sensed to the substrate surface. It is occasionally preferable that the reaction system be subjected to vibration, rotation, or temperature control in order to facilitate the binding between the substrate and the target substance to be sensed or between the target substance and the hollow nanoparticles.

An example of a specific application of the sensing tool is confirmation of the presence or absence of and quantification of the content of a protein that is present specifically in the blood of a patient of a given disease, and such application is realized in the following manner. That is, an antibody that reacts with a protein that is present specifically in the blood of a patient of a given disease or part of such antibody is bound to the substrate, and preferably bound in an aligned state with the use of a flat membrane-like array of biorecognition molecules, to allow the patent's blood to react with the substrate, the protein originated from the patient is bound to an antibody on the substrate and aligned. Thereafter, the protein originated from the patient that is bound to the substrate is recognized with the use of hollow nanoparticles that comprise the other antibody that specifically recognizes the protein of interest and present on the surfaces or enclose fluorescent, luminescent, light absorptive, or radioisotope molecules to measure the level of fluorescent, luminescent, light absorption, or radioisotope derived from the hollow nanoparticles. A sensing technique involving the use of such biosensing tool is particularly effective when the amount of the protein originated from the patient is of a trace amount of not more than 1 nmol by the reason of amplifying trace signals. Such technique is also applicable to the measurement of environmental hormones. In the case of dioxin, for example, the full length of the "AhR" protein, which is a dioxin receptor in a mammalian body or a dioxin-biding site, is bound to a metal substrate for SPR in an aligned state. A roughly purified or untreated sample obtained from the soil, river water, or mother's milk, which may be contaminated with dioxin, is allowed to react with the substrate, the substrate is washed with a dioxin-free buffer, an intranuclear protein, "ARNT," that specifically binds to the dioxin-AhR conjugate or hollow nanoparticles that comprise an antibody that reacts with dioxin bound to the surfaces thereof are allowed to react with the substrate, and the presence or absence or the quantity of dioxin in the samples are quantified based on the changes in surface plasmon.

With the use of the biosensing tool according to the present invention, environmental hormones can be assayed not only via SPR but also via a method involving QCM, a sensing method involving the use of hollow nanoparticles presenting on the surfaces or enclosing fluorescent, luminescent, or light absorptive, or radioisotope molecules, or other methods. The dioxin content in the environment is very small. In general, accordingly, pretreatment, such as extraction of dioxin from the samples and further concentration, is required for quantification, and a long period of time of several days to several weeks is required. The use of the sensing tool of the present invention brings about the effects of signal amplification. Thus, assay can be carried out without pretreatment or via more simplified pretreatment, and the assay duration can be shortened to a period of several hours to several days.

As an example of another application of the sensing tool of the present invention, samples containing the target substances to be sensed, such as proteins or nucleic acids, are electrophoresed via SDS-PAGE or agarose gel. The products are transferred electronically or osmotically to PVDF, cellulosic, or other membranes, the transferred membranes are allowed to react in a buffer containing hollow nanoparticles comprising biorecognition molecules that can specifically recognize the target substances bound thereto and presenting on the surfaces or enclosing fluorescent, luminescent, light absorptive, or radioisotope molecules, and hollow nanoparticles are bound to the target proteins, nucleic acids, or the like that had been transferred onto the membrane. Thereafter, the unbound hollow nanoparticles are washed in a buffer containing no hollow nanoparticles, and the level of fluorescent, luminescent, or light absorptive, or radioisotope molecules derived from the hollow nanoparticles remaining on the membrane is detected.

Hollow nanoparticles comprising "proteins capable of forming particles" can be obtained by allowing proteins to express in eukaryotic cells, for example. Specifically, when "proteins capable of forming particles" are allowed to express in eukaryotic cells, such proteins are expressed and accumulated on the membrane of the endoplasmic reticulum as membrane proteins, and such proteins are released as particles. In such a case, yeast, insect, or other cells can be employed as eukaryotic cells. In the present invention, yeast, animal, and insect cells include genetically recombinant yeast, animal, and insect cells. A method of forming hollow nanoparticles using yeast is preferable since particles are effectively produced from soluble proteins in the cells. On the other hand, insect cells are eukaryotic cells more similar to those of a higher-order animal compared with yeast, insect cells are preferable in the mass production of foreign proteins, in terms of the capacity for modification of a higher-order structure, such as sugar chains, that cannot be modified with yeast.

Conventional systems for forming particles with the use of insect cells involve the use of baculoviruses and involve the expression of viral particles. Accordingly, cells are likely to die or lyse upon protein expression. As a result, the expressed proteins are disadvantageously degraded by a protease released from the dead cells. When proteins are allowed to express and secrete, proteins are contaminated with a large quantity of fetal bovine serum contained in the medium, and thus, proteins secreted in the medium are often difficult to purify. In recent years, however, insect cell systems that do not involve baculoviruses or serum-free culture reagents have been sold commercially by Invitrogen. Use of such materials facilitates purification, and protein particles maintaining higher-order structures and sugar chain modification can be obtained. Subviral particles obtained from various viruses can be employed as such "proteins capable of forming particles." Specific examples thereof include surface antigens of the hepatitis B virus (HBV), hepatitis C virus, microvirus, phagevirus, adenovirus, hepasona virus, parbovirus, papovavirus, retrovirus, reovirus, coronavirus, and Bombyx mori cytoplasmic polyhedrosis virus and polyhedron proteins.

The present inventors have discovered and reported that expression of the HBV surface antigen L proteins in recombinant yeast cells resulted in the formation of large quantities of oval hollow nanoparticles each having a minor axis of approximately 20 nm and a major axis of approximately 150 nm comprising such proteins embedded therein in the lipid bilayer derived from the yeast endoplasmic reticulum from the expressed HBV surface antigen L protein, as described in the Examples below (J. Bio. Chem., Vol. 267, No. 3, 1953-1961, 1992). Since such particles are completely free of HBV genomes or other HBV proteins, they do not function as viruses, and the safety thereof in human bodies is very high. Further, production of the hollow nanoparticles of the present invention with the use of yeast does not require the use of bovine serum or the like, which may involve a risk of pathogenicity infecting human bodies. Thus, the safety thereof in human bodies can be further enhanced.

The hollow nanoparticles comprising lipids according to the present invention can specifically recognize various substances (e.g., nucleic acids, proteins, peptides, or compounds) and remarkably enhance detection sensitivity via the conversion of "proteins capable of forming particles" on the surfaces of the particles obtained by various techniques as described above into any biorecognition molecules or the binding of any biorecognition molecules to such proteins.

The "proteins capable of forming particles" are not limited to the aforementioned hepatitis B virus surface antigen proteins. Any proteins capable of forming particles can be employed. Examples of such proteins include naturally occurring proteins and genetically engineered proteins derived from animal cells, plant cells, insect cells, viruses, phages, bacteria, and fungi and various synthetic proteins. Virus-derived proteins are preferable, hepatitis virus-derived proteins are more preferable, and hepatitis B virus-derived proteins are further preferable.

An example of biorecognition molecules that are bound to the "proteins capable of forming particles" is molecules that control cellular functions. In the present invention, the term "molecules that control cellular functions" refers to factors that govern various vital phenomena necessary for cell survival. Examples thereof include antibodies, antibody analogues, antigen substances reacting with various antibodies, proteins bound to antibodies, growth factors, cytokines, enzymes, receptor proteins reacting with various ligand substances on the surfaces of or in cells, biological hormones, environmental hormones, chaperone proteins, and amyloid-forming proteins. When such substances are actually employed as biorecognition molecules, they may not necessarily comprise the full-length sequence of the aforementioned molecules; parts of such molecules or derivatives thereof may be employed. Such biorecognition molecules are composed of proteins, peptides, nucleic acids, sugar chains, or lipids. Derivatives thereof may be, for example, proteins, peptides, or nucleic acids modified with sugar chains, phosphoric acid, or a compound. Similarly, sugars or lipids modified with proteins, peptides, nucleic acids, or the like may also be employed.

The "molecules that control cellular functions" of the present invention can be obtained by, for example, a method wherein molecules are purified from living cells with the use of various types of purification columns or a method wherein a plasmid expressing the molecules that control cellular functions is prepared via genetic recombination and such molecules are produced and purified with the use of *E. coli,* yeast, insect, animal, or plant cells or a cell-free protein synthesis system. When a genetic recombination technique is employed, a tag substance, such as a His-tag, can be added to the molecules that control cellular functions for the purpose of simplifying purification. In order to easily perform the expression and purification of molecules that control cellular functions without the addition of a tag, a cell-free system, i.e., Puresystem (PGI), can be employed. When the molecules that control cellular functions as the biorecognition molecules of the present invention are not proteins (e.g., nucleic acids or steroid substances), they may be purified from an organism or chemically synthesized.

In the present invention, the term "antibody analogue" refers to an organism-derived or artificial compound that can specifically recognize an antigen substance, excluding an antibody prepared from an organism. Examples thereof include a single chain antibody or affibody obtained via selective modification of an antigen recognition site of an antibody via genetic recombination, a protein that specifically recognizes a nucleic acid molecule containing a DNA sequence that is specifically recognized by a DNA-binding protein or a given nucleic acid sequence, and a protein or compound that can specifically recognize a fibrous structure such as amyloid Such substances are adequately selected in accordance with the target substances (e.g., proteins, nucleic acids, compounds, cells, or tissues). Such antibody analogue can be obtained by a method wherein a plasmid that expresses an antibody analogue is prepared via genetic recombination, and an antibody analogue is prepared with the use of cells, such as E. coli, yeast, insect, animal, or plant cells, or with the use of a cell-free protein synthesis system, followed by purification, or a method wherein an antibody is cleaved with a protease and part of the cleavage product is purified. When an antibody analogue is not a protein, a product of chemical synthesis or an extract from a cell can be used. When a genetic recombination technique is employed, a tag substance, such as a His-tag, can be added to the antibody analogues for the purpose of simplifying purification. In order to easily perform the expression and purification of antibody analogues without the addition of a tag, a cell-free system, i.e., Puresystem (PGI), can be employed. The "biorecognition molecules" of the present invention are not limited to molecules that control cellular functions, and molecules that can specifically bind to the target substances to be sensed are sufficient.

When preparing the particles of the present invention, various applications may be adopted according to need. For example, fluorescent, luminescent, light absorptive, or radioisotope substances may be enclosed in the hollow nanoparticles via electroporation, ultrasonication, or spontaneous diffusion to amplify the sensed signals. In this case, the term "electroporation" refers to a method of introducing a substance via the application of electrical shock. The optimal conditions differ depending on the properties, concentration, or other features of the substance to be enclosed. In general, various substances can be enclosed in the particles with the application of a voltage of approximately 200 mV to 1000 V for several msec to several min. Enclosure via ultrasonication or spontaneous diffusion is realized by allowing the hollow nanoparticles and the substances to be enclosed to be present in water or a buffer and stirring the particles and the target substances, allowing the particles and the target substances to stand, or applying an ultrasonic wave at an intensity of 10 to 400 watts for 10 seconds to 2 hours. In addition to the target biorecognition molecules, a tag protein for purifying the particles, a fluorescent, luminescent, light absorptive, or radioisotope substance for facilitating sensing, or a substance for immobilizing the target substances on various types of sensing substrates (i.e., substrates used when specifically sensing substances via SPR or QCM) can be bound to part of the "protein capable of forming particles" via chemical modification or genetic engineering. In such a case, a plurality of molecules can be simultaneously bound to a single hollow nanoparticle. Such binding of a plurality of molecules to a particle surface (the exterior or interior of a particle) can be realized by allowing a plurality of expression vectors comprising different molecules bound to "proteins capable of forming particles," instead of a single expression vector, to simultaneously express in a single cell. As techniques for presenting a protein on the surface layer, a phage display method, a method of presenting a protein on a yeast surface layer, a method wherein an antibody or the like is bound to the surface of a polymer or metal particle (Szardenings M, J. Recept Signal Transduct Res., 23, 307-349, 2003; Mitsuyoshi Ueda, Bioscience and Industry, 55, 253-254, 1997; Mitsuyoshi Ueda and Toshiyuki Murai, Bioengineering, 76, 506-510, 1998), and other methods are known. These techniques, however, suffer from drawbacks in terms of safety, particle size, or ease of preparation, and the utilization of such techniques as sensing systems is limited. The nanoparticles of the present invention are purified protein particles. Thus, such particles are noninfectious and less susceptible to environmental changes via solvents and the like. From the viewpoint of handleability, the diameter is preferably 20 nm to 500 nm, and more preferably 50 nm to 200 nm. As described in the article of the present inventors, Anal. Biochem., Vol. 309, 196-119, 2002, the nanoparticles of the present invention have a structure of a flat membrane upon the spreading thereof on a silicon substrate or the like. Thus, a "flat membrane-like array of biorecognition molecules" comprising proteins bound to particle surfaces aligned on the substrate through a lipid bilayer can be formed. At the same time, this "flat membrane-like array of biorecognition molecules" covers a lipid bilayer on the substrate and it can prevent nonspecific adsorption of substances other than the target proteins or the like to the substrate.

Thus, the hollow nanoparticle-based biosensing tool according to the present invention can sense substances via the spreading of the hollow nanoparticles on the substrate, for the purpose of aligning the orientation of the proteins bound to particle surfaces or preventing nonspecific adsorption. Further, the hollow nanoparticle-based biosensing tool according to the present invention can be utilized as a sensing tool for the sandwich technique that is often employed in ELISA. That is, the target substances to be sensed are bound to the flat membrane-like array of biorecognition molecules, other hollow nanoparticles are applied thereto to detect the target substances, and the sensitivity for detecting the target substances can be improved while blocking nonspecific adsorption to the substrate. Thus, the hollow nanoparticle-based biosensing tool according to the present invention can provide sensitivity and specificity that could not be attained with the use of conventional biosensing tools through various types of modifications.

As mentioned above, in the case of the hollow nanoparticle-based biosensing tool of the present invention comprising lipids and proteins and the sensing method utilizing such sensing tool, the particles are physically very stable and fluidity is created since the particles comprise lipid membrane components on the surfaces, and nonspecific adsorption of substances can be blocked. In comparison with a method of presenting a protein on the cell or virus surface, the technique for the present invention is safer. Since the particles each have a diameter of approximately 100 nm, such particles can be easily applied to existing biosensing techniques, such as SPR, and a sensed signal of a minor level can be effectively amplified. With the use of the tool of the present invention, the signals generated when sensing trace amounts of molecules are effectively amplified. Thus, the techniques for the present invention can be applied to various fields that involve the aim of sensing trace components *in vivo* or in the environment in a simple and highly sensitive manner, such as in the case of protein chips, sensing and evaluation of diagnostic markers in medicine, measurement of environmental substances, or assay and analysis of biochemical substances.

### Examples

Hereafter, examples are presented with reference to the attached drawings, and the embodiments of the invention are described in greater detail. It should be noted that the technical scope of the present invention is not limited to the following examples and that various modifications can be made concerning the details of the techniques.

In the following example, an SPR apparatus comprising hollow nanoparticles to be bound to a metal substrate in combination with a metal substrate as described in Examples 1 and 3 and an assay apparatus for fluorescent hollow nanoparticles comprising an anti-histidine tagged 96-well plate, GFP-fused particles, and a fluorescent plate reader as described in Example 2 are used as the sensing tools according to the present invention.

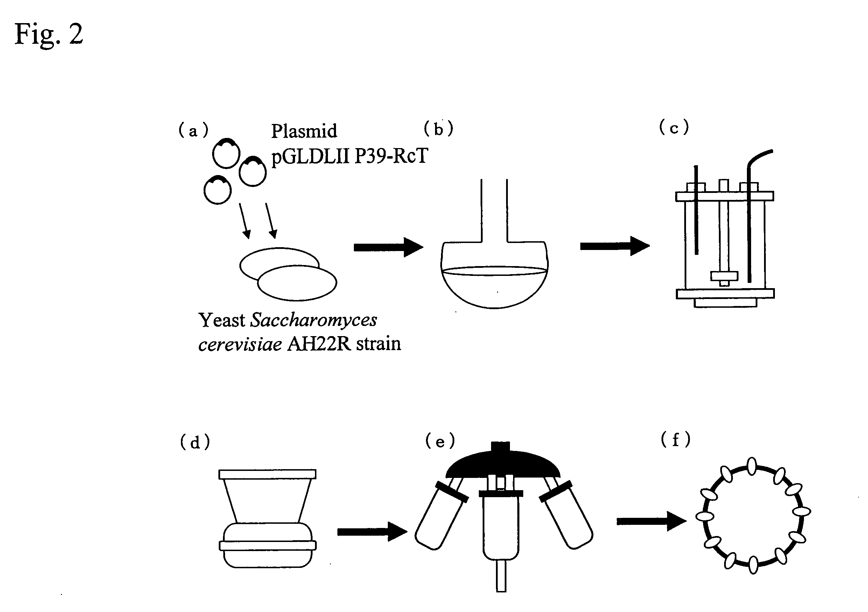

In the following example, HBsAg refers to a hepatitis B virus surface antigen. HBsAg is an HBV envelope protein. As shown in Fig. 1, HBsAg is classified into 3 types; i.e., the S protein, the M protein, and the L protein. The S protein is an important envelope protein common in the three types of proteins, the M protein has a 55 amino acid extension (pre-S2 peptide) at the N terminus compared with the S protein, and the L protein has a 108-119 amino acid extension (pre-S1 peptide) at the N terminus compared with the M protein. The pre-S regions (pre-S1 and pre-S2) of the HBsAg L protein are known to play important roles upon binding of HBV to hepatic cells. Pre-S1 has a site that directly binds to a hepatic cell and pre-S2 has a receptor for polymerized albumin that binds to a hepatic cell through polymerized albumin in the blood. When HBsAg is allowed to express in a eukaryotic cell, such protein is expressed and accumulated on the membrane of the endoplasmic reticulum as a membrane protein. The HBsAg L proteins aggregate among molecules and are released as particles to the lumen side via budding while incorporating the membrane of the endoplasmic reticulum. In the following examples, the HBsAg L protein and a variant thereof were used. Fig. 2 schematically shows examples of the expression of the HBsAg particles and the procedure for purifying the same described in the following examples.

### [Example 1]

### 1. Expression of HBsAg particles using recombinant yeast

In accordance with the method disclosed in J. Bio. Chem., Vol. 267, No. 3, 1953-1961, 1992 reported by the present inventors, a recombinant yeast strain maintaining pGLDLIIP39-RcT (*Saccharomyces Cerevisiae* AH22R-strain) was cultured in synthetic media High-Pi and 8S5N-p400 to express HBsAg L protein particles (Fig. 2a, b). The whole cell extract was prepared from a recombinant yeast strain at a stationary growth phase (approximately 72 hours after the initiation of culture) using the Yeast Protein Extraction Reagent (Pierce Chemical Co. Ltd.), the whole cell extract was subjected to separation via sodium dodecyl sulfate-polyacrylamide gel electrophoresis (SDS-PAGE), and the HBsAg protein in the sample was identified via silver staining and via Western blotting using an antibody reacting with the HBs proteins (primary antibody: anti-HBsAg monoclonal antibody).

### 2. Purification of HBsAg particles from recombinant yeast

(1) The recombinant yeast (wet weight: 26 g) that had been cultured in a synthetic medium 8S5N-P400 was suspended in 100 ml of buffer solution A (7.5 M urea, 0.1 M sodium phosphate, pH 7.2, 15 mM EDTA, 2 mM PMSF, and 0.1 % Tween 80) and the yeast was ground in a bead beater using glass beads. Thereafter, the supernatant was recovered via centrifugation (Fig. 2c, d).
(2) Subsequently, the supernatant was mixed with 0.75x volume of 33% (w/w) PEG 6000, and the mixture was kept on ice for 30 minutes. Thereafter, a pellet was recovered by centrifugation (7000 rpm, 30 minutes). The pellet was resuspended in buffer solution A containing no Tween 80.
(3) The resuspended solution was superposed on CsCl with a gradient of 10% to 40%, and ultracentrifugation was carried out at 28,000 rpm for 16 hours. The centrifuged sample was divided into 12 fractions, and an HBsAg-containing fraction was identified by Western blotting. The HBsAg-containing fraction was dialyzed in buffer solution A containing no Tween 80.
(4) The dialyzed solution (12 ml) obtained in (3) was superposed on sucrose with a gradient of 5% to 50%, and ultracentrifugation was carried out at 28,000 rpm for 16 hours. As with the case of (3), an HBsAg-containing fraction was then identified, and the HBsAg-containing fraction was dialyzed in buffer solution A containing 0.85% NaCl instead of urea and Tween 80 ((2) to (4): Fig. 2e).
(5) The same procedure as that of (4) was repeated, the dialyzed sample was concentrated using the Ultra Filter Q 2000 (Advantech), and the concentrate was refrigerated at 4°C before use (Fig. 2f). Based on the results of Western blotting (3) after CsCl equilibrium centrifugation, it was confirmed that HBsAg was a protein having a molecular weight of 52 kDa and possessing S antigenicity. Finally, approximately 24 mg of purified HBsAg particles were obtained from 26 g of cells (on a wet weight basis) in 2.5 I of medium. The fraction obtained after the purification processes was analyzed via silver staining SDS-PAGE. In order to confirm that yeast-derived protease was removed via purification, the HBsAg particles obtained in (5) were incubated at 37°C for 12 hours, subjected to SDS-PAGE, and then subjected to identification via silver staining. As a result, it was found that the yeast-derived protease had been completely removed through the purification process.

### 3. Amplification of surface plasmon signal by nanoparticles

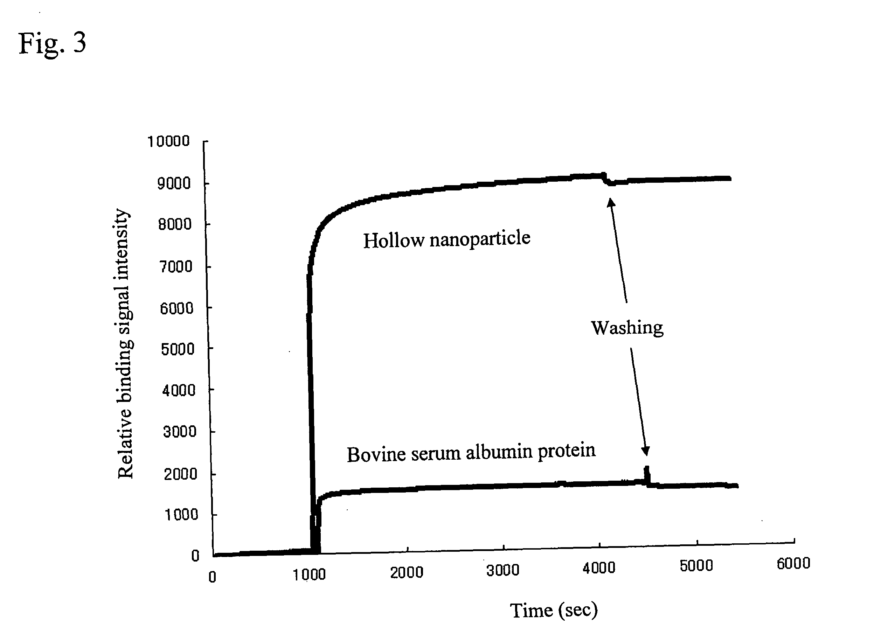

All SPR procedures were carried out at 25°C. The HBsAg particles purified from yeast in the above-described manner were allowed to adsorb to an SPR assay chip for Nanosensor (the Nippon Laser Electronics & Lab.) at 50 mg/ml. As a control sample, bovine serum albumin (BSA) protein was allowed to adsorb to a chip in the same manner, and changes in the signal generated when the proteins were bound to the SPR metal substrate were assayed. As a result, the SPR signal generated with the use of the HBsAg particles was as high as 6 times higher than that generated with the use of BSA, as shown in Fig. 3. The SPR signals of various proteins, such as GFP or IgG proteins, were compared in the same manner, and consequently, the SPR signal, generated with binding of the HBsAg of the HBsAg particle to SPR substrate, was amplified to a level 5 to 10 times higher than that generated with the use of the aforementioned protein. This demonstrates that the biosensing method involving the use of the nanoparticles of the present invention can dramatically improve detection sensitivity.

### [Example 2]

### 1. Preparation of expression vector of HBsAg L particle fused with biorecognition protein via genetic recombination

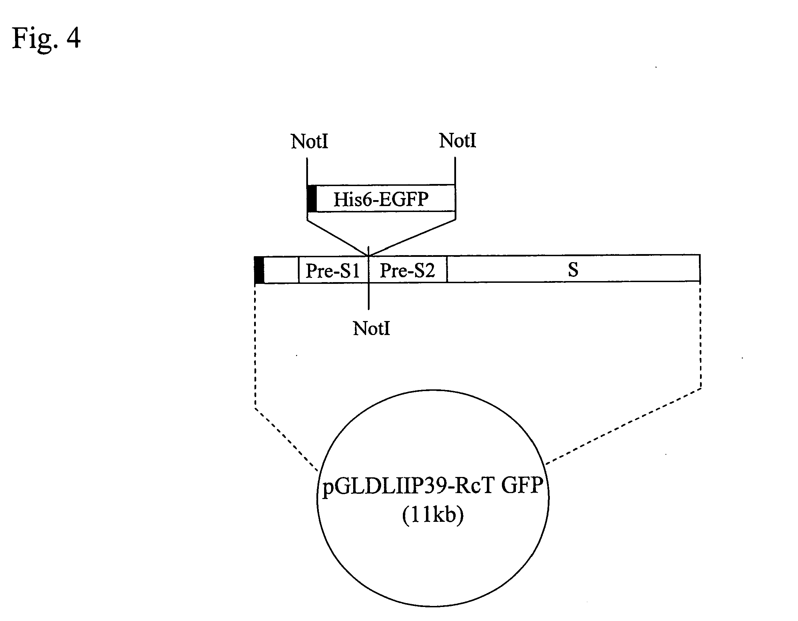

At the outset, as described in JP Patent Publication (Kokai) No. 2001-316298 A of the present inventors, pGLDLIIP39-RcT, which was prepared in accordance with the method disclosed in the report of the present inventors, i.e., J. Bio. Chem., Vol. 267, No. 3, 1953-1961, 1992, was modified to prepare the "expression vector of the HBsAg L particles fused with biorecognition proteins" of Example 2; i.e., pGLDLIIP39-RcT-GFP. The method for preparing the same is briefly described below. With the use of primers as shown in SEQ ID NOs: 1 and 2, pGLDLIIP39-RcT null was first prepared by substituting a part of the L protein sequence (a 3-77 coding region) of HBsAg existing on the pGLDLIIP39-RcT with the sequence of the *Not*I restriction enzyme site (gcggccgc). This substitution was carried out using the QuikChange® Site-Directed Mutagenesis Kit (Stratagene) in accordance with the protocol of the kit. The primers as shown in SEQ ID NOs: 1 and 2 are of complementary sequences designed in accordance with the protocol of the aforementioned kit for conducting such substitution. Subsequently, PCR was carried out using the primers as shown in SEQ ID NOs: 3 and 4 to amplify the GFP gene. The primer as shown in SEQ ID NO: 3 has a sequence of the *Not*I restriction enzyme site at the 5' end, a sequence encoding 6 continuous histidine residues, and a sequence recognizing 29 nucleotides of the 5' end of the GFP gene downstream of the restriction enzyme site. The primer as shown in SEQ ID NO: 4 has a sequence of the *Not*I restriction enzyme site at the 5' end and a sequence recognizing 20 nucleotides of the 3' end of the GFP gene downstream thereof. The PCR product amplified with the use of these primers as shown in SEQ ID NOs: 3 and 4 was treated with the *Not*I restriction enzyme and inserted into and ligated to the *Not*I-cleaved vector, pGLDLIIP39-RcT null, prepared above. Thus, a vector, pGLDLIIP39-RcT-GFP, comprising GFP having a histidine tag at the N-end inserted into pGLDLIIP39-RcT null was prepared (Fig. 4).

### 2. Expression of HBsAg L particles fused with biorecognition proteins in recombinant yeast

The recombinant yeast strain (*Saccharomyces Cerevisiae* AH22R-strain) maintaining the yeast expression vector pGLDLIIP39-RcT-GFP was cultured in synthetic media High-Pi and 8S5N-p400 to express HBsAg L protein particles fused with biorecognition proteins (Fig. 2a, b). The whole cell extract was prepared from a recombinant yeast strain at a stationary growth phase (approximately 72 hours after the initiation of culture) using the Yeast Protein Extraction Reagent (Pierce Chemical Co. Ltd.), the whole cell extract was subjected to separation via sodium dodecyl sulfate-polyacrylamide gel electrophoresis (SDS-PAGE), and the biorecognition proteins fused with the HBsAg L proteins in the sample were identified via silver staining and via Western blotting using an antibody reacting with a histidine tag. pGLDLIIP39-RcT-GFP was transformed into a yeast strain AH22R in accordance with the method reported by the present inventors, which is disclosed in JP Patent Publication (Kokai) No. 2001-316298 A. This demonstrates that the HBsAg L protein fused with biorecognition proteins (fusion of GFP) had a molecular weight of 72 kDa.

### 3. Purification of HBsAg particles from recombinant yeast

(1) The recombinant yeast (wet weight: 10 g) that had been cultured in 1 I of a synthetic medium 8S5N-P400 was suspended in 100 ml of buffer solution A (0.1 M sodium phosphate, pH 7.2, 15 mM EDTA, 2 mM PMSF, and 0.1 % Tween 80) and the yeast was ground in a bead beater using glass beads having an average diameter of 0. 5 mm. Thereafter, the supernatant was recovered via centrifugation (Fig. 2c, d).
(2) Subsequently, the supernatant was purified based on histidine-nickel (Ni2+) affinity using the 1-ml HisTrap column (Amersham) and a protein purification apparatus (ACTA primer, Amersham). Purification was carried out by allowing a histidine tag to bind to a nickel column in a PBS(-) buffer (Daiichi Pharmaceutical Co., Ltd.) containing 60 mM imidazole, washing the column with 20 ml of the buffer, and extracting the particles bound to nickel using a PBS(-) buffer containing 500 mM imidazole. The extracted protein particles were subjected to silver staining and Western blotting with the use of an antibody reacting with a histidine tag to confirm the degree of purification.
(3) Subsequently, the proteins were subjected to desalting and concentration using an ultrafilter membrane having 100 kDa cutoff molecular weight (Ultra Filter Q2000 (Advantech), and the resultant was refrigerated at 4°C before use.
(4) The purified HBsAg particles fused with GFP were found to emit intense green fluorescence at 510 nm under a fluorescence microscope (BX51, Olympus).
(5) As a consequence of the purification procedure, approximately 2 mg of purified particles were obtained from 10 g of cells (on a wet weight basis) in 1 1 of medium.

### 4. Sensing of anti-histidine tag antibody using GFP-fused particles

The target substances to be sensed, anti-histidine tag antibodies at various concentrations (anti-His6 monoclonal antibodies, Nacalai Tesque), were allowed to react with (bind to) a 96-well plate at 4°C for 16 hours. Thereafter, the plates were blocked with 0.05% bovine serum albumin at room temperature for 1 hour. Subsequently, 200 ml of a solution containing the GFP-fused particles (concentration: 50 mg/ml) that was purified from yeast in the manner described in Example 1 were added thereto, the reaction was allowed to proceed at room temperature, unreacted particles were washed three times with PBS, and the fluorescent level of GFP bound to the anti-histidine tag antibody was assayed using a fluorescence plate reader (Spectra Max Gemini EM, Molecular device) at 484 nm with extension at 510 nm with emission. As a control sample, a histidine-tagged GFP protein purified from *E. coli* was assayed under the same conditions. As a result, the GFP-fused particles were found to bind to an anti-histidine tag antibodies with higher sensitivity and detection could be carried out with sensitivity 100 times higher than that of the control GFP sample (Table 1).

**Table 1**

| Concentration of anti-histidine tag antibody (µg/ml) | 0 | **1.56** | **3.13** | 6.25 | 12.5 | 25 | 50 | 100 | **200** |
|---|---|---|---|---|---|---|---|---|---|
| GFP-fused particles | 12 | **37** | **65** | 111 | 217 | **447** | 702 | 966 | 1,329 |
| His-tagged GFP | 12 | 14 | 14 | 15 | 16 | 22 | 29 | 39 | **51** |

### [Example 3]

### Sensing of histidine tag via SPR

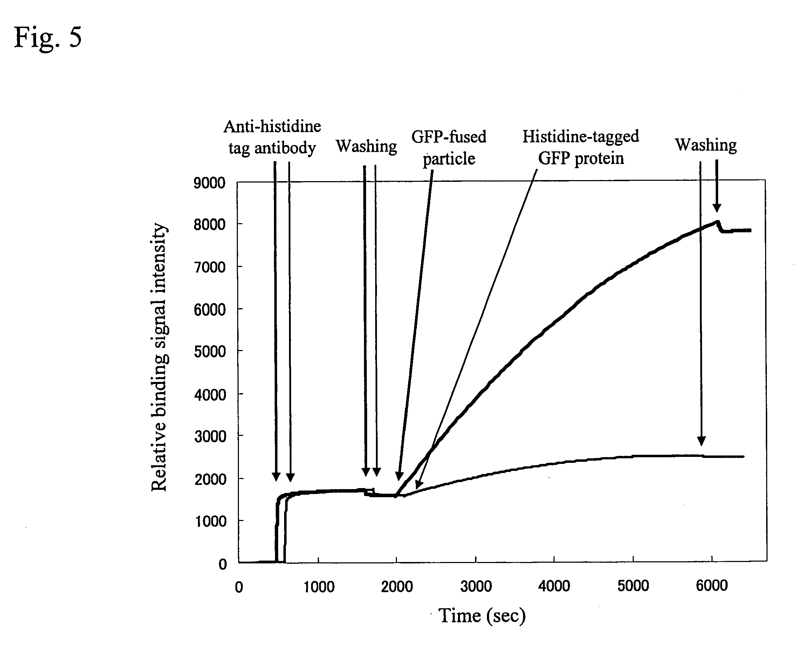

The SPR assay chip to which the anti-histidine tag antibodies were bound at 50 mg/ml used in Example 2 was mounted on the Nanosensor (the Nippon Laser Electronics & Lab.), the GFP-fused particles purified from yeast in the manner described in Example 1 and control histidine-tagged GFP proteins were allowed to simultaneously flow over the same, and the detection sensitivity of the histidine tag on the nickel substrate was assayed. As shown in Fig. 5, the sensitivity of a histidine tag for sensing the anti-histidine tag antibodies via SPR was found to be significantly improved compared with that of the control experiment using the same amount of antibodies. This was also confirmed by the amino coupling method involving the use of a nickel complex bound to the SPR sensor instead of anti-histidine tag antibodies.

### [Example 4]

### Sensing of anti-histidine tag antibody via SPR

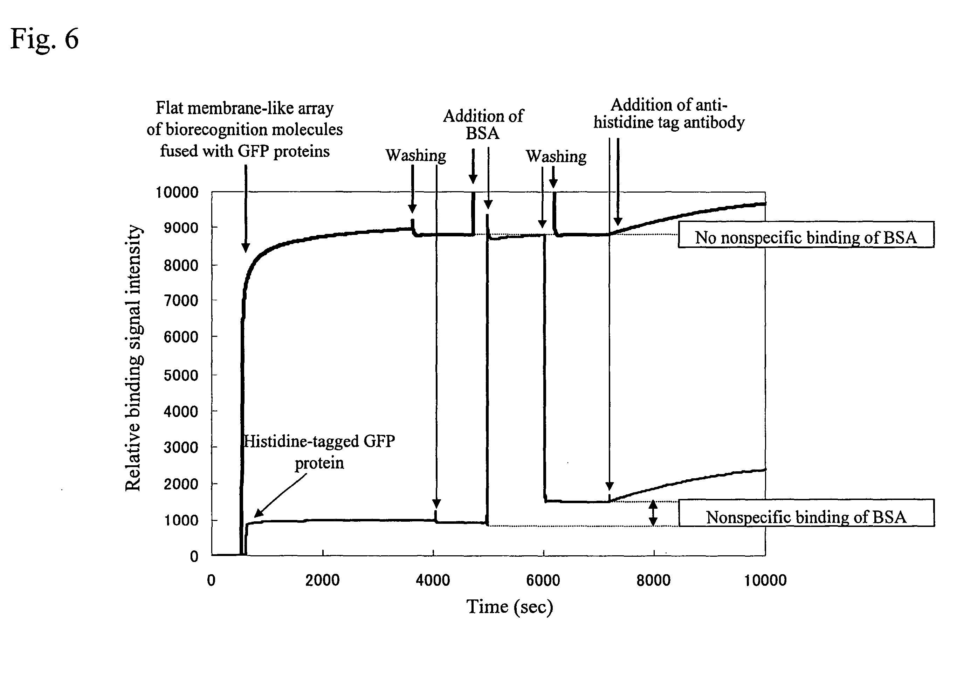

The histidine-tagged GFP-fused particles described in Example 2 were bound to the SPR assay chip for Nanosensor (the Nippon Laser Electronics & Lab.) at 1 mg/ml to form a flat membrane-like array of biorecognition molecules using the GFP-fused particles. After the unreacted GFP-fused particles were washed with PBS, a 2 mg/ml BSA solution was applied to the assay chip, and reaction was allowed to proceed for 10 minutes, followed by washing with PBS. Thus, nonspecific binding of BSA to the flat membrane-like array of biorecognition molecules was inspected. Thereafter, a 200 µg/ml anti-histidine tag antibodies was applied to the assay chip and the reaction was allowed to proceed for 10 minutes. As a control sample that would not form a flat membrane-like array of biorecognition molecules, the histidine-tagged GFP proteins purified from *E. coli* were allowed to bind to the SPR assay chip at 1 mg/ml, and the same experiment was carried out. The results are shown in Fig. 6.

When the histidine-tagged GFP proteins purified from *E. coli* were bound to the SPR assay chip, nonspecific binding of BSA was observed. On the contrary, nonspecific binding of BSA was not observed with the use of the flat membrane-like array of biorecognition molecules formed by allowing the GFP-fused particles to bind to the SPR assay chip. When the anti-histidine tag antibodies were applied to the flat membrane-like array of biorecognition molecules, a stable signal was observed due to specific binding caused by antigen-antibody reactions. Thus, the flat membrane-like array of biorecognition molecules comprising the hollow nanoparticles of the present invention were found to block nonspecific binding, have aligned biorecognition molecules, and be suitable for detection that would require high sensitivity.

All publications, patents, and patent applications cited herein are incorporated herein by reference in their entirety.

### Industrial Applicability

The present invention provides a versatile tool for effectively detecting trace components *in vivo* or in the environment that utilizes hollow nanoparticles presenting biorecognition molecules and a sensing technique using such tool. The sensing tool and the sensing method of the present invention can be applied to various fields that require the detection of trace components, such as in the case of protein chips, sensing and evaluation of diagnostic markers in medicine, measurement of environmental substances, or assay and analysis of biochemical substances.

### Free Text of Sequence Listings

SEQ ID NO: 1- description of artificial sequence: a primer for the site-directed mutagenesis on pGLDLIIP39-RcT by QuikChange® Site-Directed Mutagenesis
SEQ ID NO: 2- description of artificial sequence: primer for the site-directed mutagenesis on pGLDLIIP39-RcT by QuikChange® Site-Directed Mutagenesis
SEQ ID NO: 3- description of artificial sequence: primer for the GFP amplification by PCR, including six consecutive histidine codon sequence
SEQ ID NO: 4- description of engineered sequence: a primer for amplification by PCR

## Claims

1. A sensing tool comprising proteins capable of forming nanoparticles through the incorporation of a lipid bilayer and biorecognition molecules bound thereto.

2. The sensing tool according to claim 1, wherein the biorecognition molecules are covalently bound to the proteins.

3. The sensing tool according to claim 1, wherein the nanoparticles are hollow nanoparticles.

4. The sensing tool according to claim 1, wherein the proteins are virus surface antigen proteins.

5. The sensing tool according to claim 1, wherein the proteins are hepatitis B virus surface antigen proteins.

6. The sensing tool according to claim 1, wherein the proteins are capable of forming nanoparticles through the incorporation of a lipid bilayer derived from eukaryotic cells.

7. The sensing tool according to claim 1, wherein the proteins are capable of forming nanoparticles through the incorporation of a lipid bilayer derived from yeast.

8. The sensing tool according to claim 1, wherein the proteins are capable of forming nanoparticles through the incorporation of a lipid bilayer derived from animal or insect cells.

9. The sensing tool according to claim 1, wherein the biorecognition molecules are molecules that control cellular functions.

10. The sensing tool according to claim 1, wherein the biorecognition molecules are antigens, antibodies, parts of antibodies, or antibody analogues.

11. The sensing tool according to claim 1, wherein the biorecognition molecules are cell surface or intracellular receptor proteins that bind to ligand substances, mutants thereof, parts thereof, or substances bound thereto.

12. The sensing tool according to claim 1, wherein the biorecognition molecules are enzymes, mutants thereof, parts thereof, or substances bound thereto.

13. The sensing tool according to claim 1, wherein at least one type of molecule selected from the group consisting of fluorescent, luminescent, light absorptive, and radioisotope molecules is bound to the biorecognition molecules.

14. The sensing tool according to claim 1, wherein at least one type of molecule selected from the group consisting of fluorescent, luminescent, light absorptive, and radioisotope molecules is bound to proteins capable of forming particles.

15. The sensing tool according to claim 1, wherein at least one type of molecule selected from the group consisting of fluorescent, luminescent, light absorptive, and radioisotope molecules is bound to the lipid bilayer.

16. The sensing tool according to claim 1, wherein at least one type of molecule selected from the group consisting of fluorescent, luminescent, light absorptive, and radioisotope molecules is enclosed in hollow nanoparticles.

17. The sensing tool according to claim 1, which employs a flat membrane-like array of biorecognition molecules comprising nanoparticles aligned on a substrate.

18. A biosensing method, which involves the use of the sensing tool according to claim 1.
